# EUROPEAN PATENT APPLICATION

(11) **EP 3 543 689 A1**
(43) Date of publication of application: **25.09.2019**
(21) Application number: 17892540.0
(22) Date of filing: 25.12.2017
(51) Int. Cl.: G01N 29/24

(54) **ULTRASONIC PROBE**

(30) Priority: 19.01.2017 JP 2017007432
(71) Applicant: Kabushiki Kaisha Kobe Seiko Sho (Kobe Steel, Ltd.), Kobe-shi, Hyogo 651-8585 (JP)
(72) Inventor: FUKUI, Toshihide, Hyogo 651-2271 (JP); WASA, Yasuhiro, Hyogo 651-2271 (JP); NOMURA, Kota, Hyogo 651-2271 (JP)
(74) Representative: Müller-Boré & Partner Patentanwälte PartG mbB
(86) International application number: PCT/JP2017/046356
(87) International publication number: WO 2018/135244

(57) **Abstract**

An ultrasonic probe comprises a transmitting transducer, a receiving transducer, an absorber, a transmitting and receiving transducer including a transmitting unit and a receiving unit, and a wedge. The wedge includes a first holding part holding the transmitting transducer, a second holding part holding the receiving transducer, an absorber holding part disposed between the first holding part and the second holding part and holding the absorber, and a third holding part disposed on the opposite side of the first holding part from the absorber holding part and holding the transmitting and receiving transducer at an angle allowing ultrasound transmitted from the transmitting and receiving transducer to propagate as surface waves along a surface area of a test object.

## Description

### Technical Field

The present invention relates to an ultrasonic probe for inspecting a test object for defects.

### Background Art

Conventional methods for detecting internal defects using ultrasound include a vertical flaw detection method and an oblique flaw detection method. Patent Literature 1 discloses a dual transducer probe including separate transmitting and receiving transducers for performing transmission and reception independently of each other.

Such separation makes it possible to reduce the surface dead zone, but still leaves a dead zone of about 1 mm. It is therefore difficult to realize inspection from the very top surface.

### Citation List

### Patent Literature

Patent Literature 1: Japanese Unexamined Patent Publication No. 2003-302388

### Summary of Invention

It is an object of the present invention to provide an ultrasonic probe that leaves almost no surface dead zone.

An ultrasonic probe according to an aspect of the present invention comprises: a transmitting transducer for transmitting ultrasound; a receiving transducer for receiving an ultrasonic echo produced by ultrasound transmitted from the transmitting transducer being reflected by a defect present in an internal area of a test object; an absorber for absorbing ultrasound transmitted from the transmitting transducer; a transmitting and receiving transducer including a transmitting unit for transmitting ultrasound and a receiving unit for receiving an ultrasonic echo produced by ultrasound transmitted from the transmitting unit being reflected by a defect present in a surface area of the test object; and a wedge holding the transmitting transducer, the receiving transducer, the absorber, and the transmitting and receiving transducer, wherein the wedge includes a first holding part holding the transmitting transducer at an angle allowing the ultrasound transmitted from the transmitting transducer to penetrate into the test object, a second holding part holding the receiving transducer at an angle allowing the receiving transducer to receive the ultrasonic echo produced by the ultrasound transmitted from the transmitting transducer being reflected by the defect present in the internal area, an absorber holding part disposed between the first holding part and the second holding part and holding the absorber, and a third holding part disposed on the opposite side of the first holding part from the absorber holding part and holding the transmitting and receiving transducer at an angle allowing the ultrasound transmitted from the transmitting and receiving transducer to propagate as surface waves along the surface area of the test object.

### Brief Description of Drawings

Fig. 1 is a schematic diagram of an ultrasonic probe according to a first embodiment of the present invention.
Fig. 2 illustrates reception signals received by a receiving transducer when a broadband transducer is used as a transmitting transducer.
Fig. 3 illustrates reception signals received by the receiving transducer when a narrow-band transducer is used as the transmitting transducer.
Fig. 4 is a schematic diagram of an ultrasonic probe according to a second embodiment of the present invention.
Fig. 5 is a schematic diagram of an ultrasonic probe according to a third embodiment of the present invention.
Fig. 6 is a schematic diagram of an ultrasonic probe according to a fourth embodiment of the present invention.

### Description of Embodiments

### (First Embodiment)

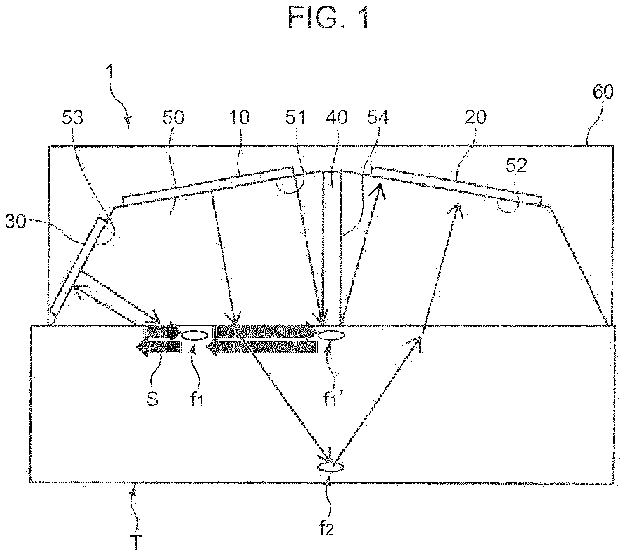

An ultrasonic probe 1 according to a first embodiment of the present invention will be described with reference to Fig. 1. The ultrasonic probe 1 can detect defects f1 and f1' present on the surface or near-surface (hereinafter, referred to as "surface area") of a test object T, such as a steel material, and can also detect a defect f2 present in an internal area of the test object T deeper than the surface area. The surface area refers to an area with a depth equal to about once or twice the wavelength of surface waves S from the surface of the test object T. Specifically, the ultrasonic probe 1 includes a transmitting transducer 10, a receiving transducer 20, a transmitting and receiving transducer 30, an absorber 40 for absorbing ultrasound, a wedge 50, and a casing 60 housing the transducers 10, 20, and 30, the absorber 40, and the wedge 50.

The transmitting transducer 10 transmits ultrasound. Specifically, the transmitting transducer 10 is preferably in the form of a broadband transducer (transducer for generating an ultrasonic pulse containing one or two cycles). The receiving transducer 20 receives ultrasound. The transmitting and receiving transducer 30 includes a transmitting unit for transmitting ultrasound and a receiving unit for receiving an ultrasonic echo produced by reflection of the ultrasound transmitted from the transmitting unit.

The wedge 50 holds the transducers 10, 20, and 30 and the absorber 40. Specifically, the wedge 50 includes a first holding part 51 holding the transmitting transducer 10, a second holding part 52 holding the receiving transducer 20, a third holding part 53 holding the transmitting and receiving transducer 30, and an absorber holding part 54 holding the absorber 40.

The first holding part 51 holds the transmitting transducer 10 at an angle allowing the ultrasound transmitted from the transmitting transducer 10 to penetrate into the test object T.

The second holding part 52 holds the receiving transducer 20 at an angle allowing the receiving transducer 20 to receive an ultrasonic echo produced by ultrasound transmitted from the transmitting transducer 10 being reflected by the defect f2 present in the internal area of the test object T. Thus, the transmitting transducer 10 and the receiving transducer 20 are used to detect the defect f2 present in the internal area of the test object T.

The absorber holding part 54 is disposed between the first holding part 51 and the second holding part 52 and holds the absorber 40. The absorber 40 prevents the ultrasound transmitted from the transmitting transducer 10 into the wedge 50 from reaching the receiving transducer 20 without passing through the test object T.

The third holding part 53 is disposed on the opposite side of the first holding part 51 from the absorber holding part 54 (on the left side in Fig. 1) and holds the transmitting and receiving transducer 30 at an angle allowing the ultrasound transmitted from the transmitting unit of the transmitting and receiving transducer 30 to propagate as surface waves S (in the form of Rayleigh waves or SH waves) along the surface area of the test object T. The receiving unit of the transmitting and receiving transducer 30 receives ultrasonic echoes produced by surface waves S being reflected by the defects f1 and f1' present in the surface area of the test object T. Thus, the transmitting and receiving transducer 30 is used to detect the defects f1 and f1' present in the surface area of the test object T.

As described above, the ultrasonic probe 1 according to the first embodiment can detect the defects f1 and f1' present in the surface area of the test object T by means of the transmitting and receiving transducer 30, and can also detect the defect f2 present in the internal area of the test object T by means of the transmitting transducer 10 and the receiving transducer 20. Thus, the present ultrasonic probe 1 leaves almost no surface dead zone.

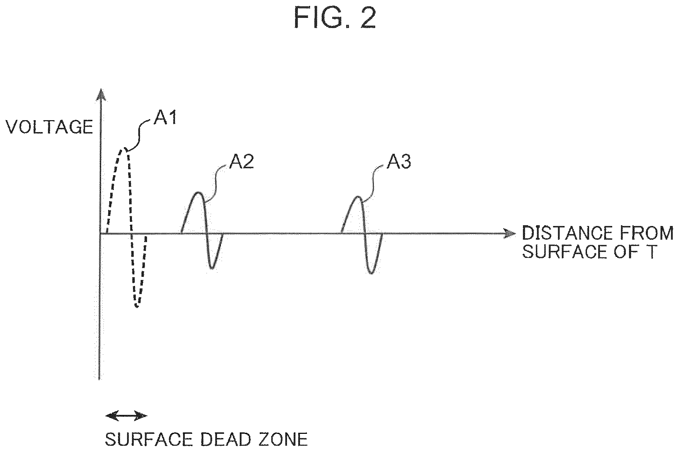

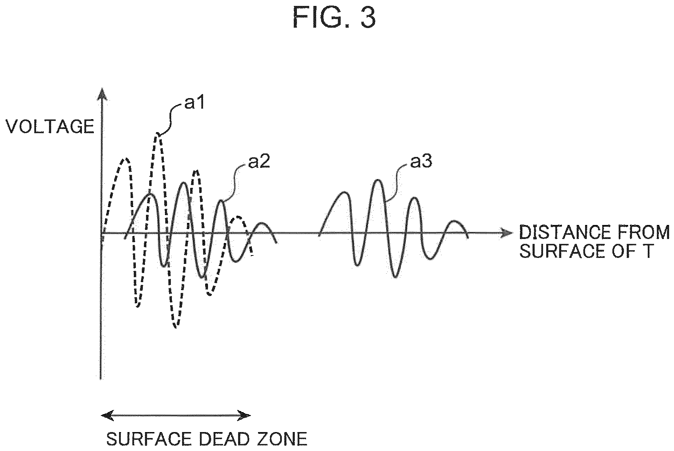

In addition, the use of broadband transducer as the transmitting transducer 10 makes it possible to detect, by means of the transmitting transducer 10 and the receiving transducer 20, the defect f1' present in a portion (directly under the absorber 40) of the part of the surface area of the test object T covered by the wedge 50 (directly under the wedge 50). Specifically, since the number of cycles of the ultrasound transmitted from the broadband transducer is small, it is possible to discriminate between a reception signal A1 that indicates reception by the receiving transducer 20 of an ultrasonic echo produced by reflection of the ultrasound on the surface of the test object T and a reception signal A2 that indicates reception by the receiving transducer 20 of an ultrasonic echo produced by reflection of the ultrasound by the defect f1', as shown in Fig. 2. In contrast, when a narrow-band transducer is used as the transmitting transducer 10, it is difficult to discriminate between a reception signal a1 that indicates reception by the receiving transducer 20 of an ultrasonic echo produced by reflection of the ultrasound on the surface of the test object T and a reception signal a2 that indicates reception by the receiving transducer 20 of an ultrasonic echo produced by reflection of the ultrasound by the defect f1', as shown in Fig. 3. In either case, it is possible to clearly discriminate reception signals A3 or a3, which indicate reception by the receiving transducer 20 of an ultrasonic echo produced by reflection of the ultrasound by the defect f2, from the other reception signals.

Further, the absorber 40, which is used to prevent the ultrasound transmitted from the transmitting transducer 10 from reaching the receiving transducer 20 without passing through the test object T, also absorbs ultrasound transmitted from the transmitting and receiving transducer 30 into the wedge 50. This makes it possible to prevent an ultrasonic echo (noise) produced by reflection of the ultrasound in the wedge 50 from being received by the transmitting and receiving transducer 30.

In contrast to conventional methods, the ultrasonic probe 1 according to the first embodiment detects the defects f1 and f1' present in the part of the surface area of the test object T covered by the wedge 50 (directly under the wedge). This reduces ultrasonic echoes (noise) caused by a couplant applied on the surface of the test object T. Consequently, the accuracy of detecting the defects f1 and f1' present in the surface area of the test object T improves. The same applies to the embodiments described below.

The use of the absorber 40 further improves the detection accuracy of the defects f1 and f1' as shown in Fig. 1. Specifically, in the present inspection method, ultrasonic echoes are produced at the defects f1 and f1' before the surface waves S reach the absorber holding part 54 of the wedge 50, and thus the absorber 40 hardly affects the production of the ultrasonic echoes caused by the defects f1 and f1'. However, in the case of a conventional method of detecting a defect present in front of the wedge in the propagation direction of surface waves, use of a wedge including an absorber would reduce the surface waves propagating beyond the wedge. This would lead to a considerable reduction of ultrasonic echoes produced at the defect. Thus, the use of the wedge 50 including the absorber holding part 54 is particularly effective in the present inspection method of detecting the defects f1 and f1' present in the part directly under the wedge 50. The same applies to the embodiments described below.

### (Second Embodiment)

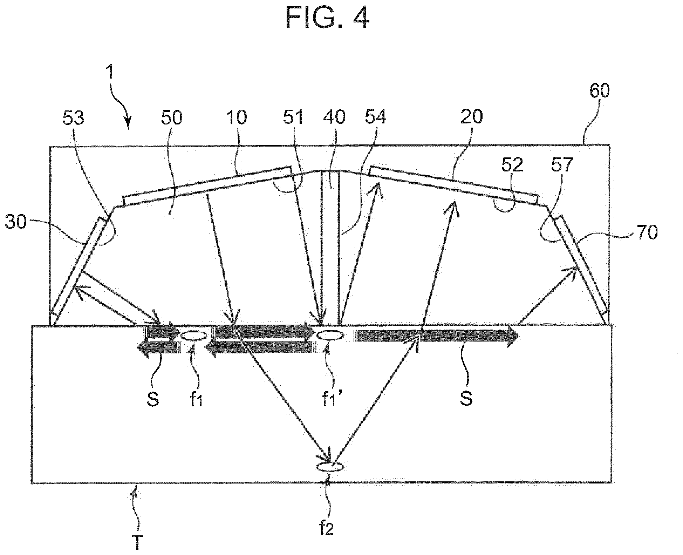

Now, a second embodiment of the present invention will be described with reference to Fig. 4. In the second embodiment, description will be made only on features different from those of the first embodiment, and thus the descriptions of structures, operations, and effects that are the same as those of the first embodiment will be omitted.

An ultrasonic probe 1 according to the second embodiment further includes a surface wave receiving transducer 70, and a fourth holding part 57 holding the surface wave receiving transducer 70 provided in a wedge 50.

The surface wave receiving transducer 70 receives ultrasound. The fourth holding part 57 is disposed on the opposite side of a second holding part 52 from the absorber holding part 54 and holds the surface wave receiving transducer 70 at an angle allowing the surface wave receiving transducer 70 to receive ultrasound produced by propagation of surface waves S into the wedge 50.

In the second embodiment, since the surface wave receiving transducer 70 is disposed on the opposite side of the second holding part 52 from the absorber holding part 54, it is possible to make sure that the wedge 50 is in contact with a test object T. Specifically, if the bottom surface between an absorber 40 and the second holding part 52 of the wedge 50 is spaced apart from the test object T, the surface wave receiving transducer 70 receives little ultrasound based on the surface waves S. Thus, reception of ultrasound by the surface wave receiving transducer 70 allows determination that the wedge 50 is in contact with the test object T.

### (Third Embodiment)

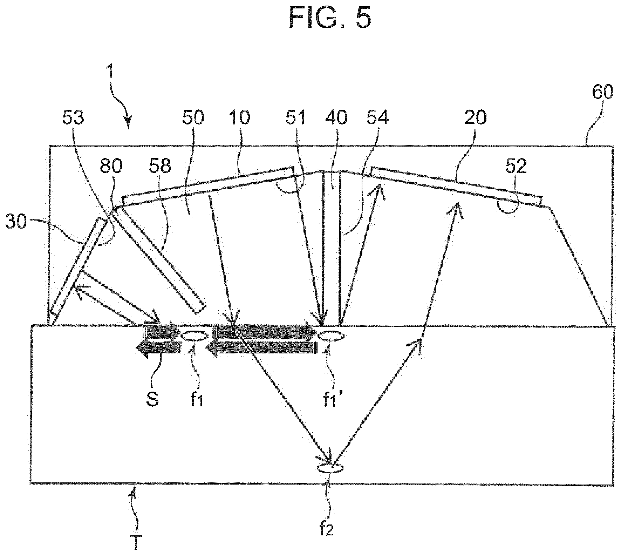

Now, a third embodiment of the present invention will be described with reference to Fig. 5. In the third embodiment, description will be made only on features different from those of the first embodiment, and thus the descriptions of structures, operations, and effects that are the same as those of the first embodiment will be omitted.

An ultrasonic probe 1 according to the third embodiment further includes a partition absorber 80, and a partition absorber holding part 58 holding the partition absorber 80 provided in a wedge 50.

The partition absorber 80 absorbs ultrasound. The partition absorber 80 is in the form of a flat plate. The partition absorber 80 is made of cork, for example. The partition absorber holding part 58 is disposed between a transmitting transducer 10 and a transmitting and receiving transducer 30 and holds the partition absorber 80. This allows the partition absorber 80 to absorb part of the respective ultrasound waves transmitted from the transmitting transducer 10 and the transmitting and receiving transducer 30 into the wedge 50. The partition absorber 80 is spaced apart from the bottom surface of the wedge 50.

In the third embodiment, the use of the partition absorber 80 makes it possible to reduce undesired ultrasonic echoes (noise) produced by the respective ultrasound waves transmitted from the transmitting transducer 10 and the transmitting and receiving transducer 30 being partially reflected in the wedge 50. This improves the accuracy of detecting defects f1 and f1' by means of the transmitting and receiving transducer 30.

In addition, since the partition absorber 80 is spaced from the bottom surface of the wedge 50, the partition absorber 80 does not block the propagation of surface waves S. This makes it possible to effectively detect the defects f1 and f1' in the surface area by means of the transmitting and receiving transducer 30.

The third embodiment may also include a surface wave receiving unit 70 and a fourth holding part 57 provided in the wedge 50, similarly to the second embodiment.

### (Fourth Embodiment)

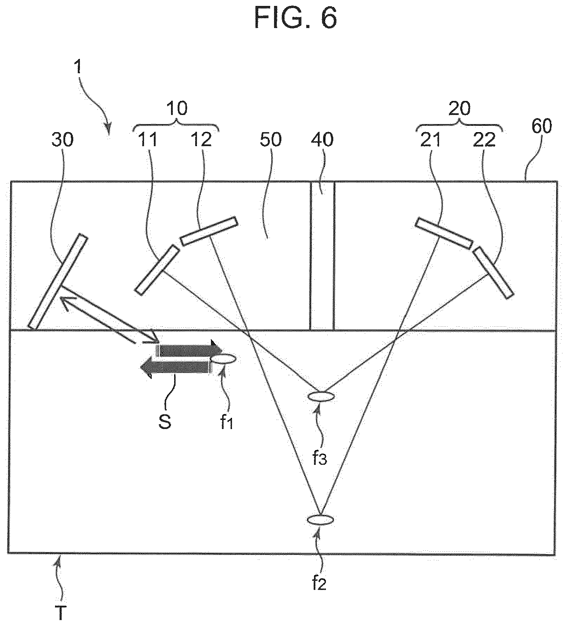

Now, a fourth embodiment of the present invention will be described with reference to Fig. 6. In the fourth embodiment, description will be made only on features different from those of the first embodiment, and thus the descriptions of structures, operations, and effects that are the same as those of the first embodiment will be omitted.

In an ultrasonic probe 1 according to the fourth embodiment, a transmitting transducer 10 includes a first transmitting unit 11 and a second transmitting unit 12 for independently transmitting ultrasound waves, and a receiving transducer 20 includes a first receiving unit 21 and a second receiving unit 22 for independently receiving ultrasound waves. A wedge 50 holds the transmitting units 11 and 12 such that the respective ultrasound waves transmitted from the first transmitting unit 11 and the second transmitting unit 12 into the wedge 50 to enter a test object T at different incident angles. In addition, the wedge 50 holds the first receiving unit 21 at an angle allowing the first receiving unit 21 to receive an ultrasonic echo produced by ultrasound transmitted from the first transmitting unit 11 being reflected by a defect f2 present in an internal area of the test object T, and also holds the second receiving unit 22 at an angle allowing the second receiving unit 22 to receive an ultrasonic echo produced by ultrasound transmitted from the second transmitting unit 12 being reflected by a defect f3 present in an internal area of the test object T.

The fourth embodiment makes it possible to effectively detect the defects f2 and f3 present at different depths in the test object T.

The described embodiments will now be summarized.

An ultrasonic probe according to the above-described embodiments comprises: a transmitting transducer for transmitting ultrasound; a receiving transducer for receiving an ultrasonic echo produced by ultrasound transmitted from the transmitting transducer being reflected by a defect present in an internal area of a test object; an absorber for absorbing ultrasound transmitted from the transmitting transducer; a transmitting and receiving transducer including a transmitting unit for transmitting ultrasound and a receiving unit for receiving an ultrasonic echo produced by ultrasound transmitted from the transmitting unit being reflected by a defect present in a surface area of the test object; and a wedge holding the transmitting transducer, the receiving transducer, the absorber, and the transmitting and receiving transducer, wherein the wedge includes a first holding part holding the transmitting transducer at an angle allowing the ultrasound transmitted from the transmitting transducer to penetrate into the test object, a second holding part holding the receiving transducer at an angle allowing the receiving transducer to receive the ultrasonic echo produced by the ultrasound transmitted from the transmitting transducer being reflected by the defect present in the internal area, an absorber holding part disposed between the first holding part and the second holding part and holding the absorber, and a third holding part disposed on the opposite side of the first holding part from the absorber holding part and holding the transmitting and receiving transducer at an angle allowing the ultrasound transmitted from the transmitting and receiving transducer to propagate as surface waves along the surface area of the test object.

The present ultrasonic probe makes it possible to provide an ultrasonic probe that leaves almost no surface dead zone.

The above-described ultrasonic probe preferably further comprises a surface wave receiving transducer for receiving ultrasound produced by propagation of the surface waves into the wedge, wherein the wedge further includes a fourth holding part disposed on the opposite side of the second holding part from the absorber holding part and holding the surface wave receiving transducer.

Such a configuration makes it possible to make sure that the wedge is in contact with the test object. Specifically, if the bottom surface between the absorber and the second holding part of the wedge is spaced apart from the test object, the surface wave receiving transducer receives little ultrasound based on the surface waves. Thus, reception of ultrasound by the surface wave receiving transducer allows determination that the wedge is in contact with the test object.

Further, the above-described ultrasonic probe preferably further comprises a partition absorber, disposed between the transmitting transducer and the transmitting and receiving transducer, for absorbing part of the respective ultrasound waves transmitted from the transmitting transducer and the transmitting and receiving transducer.

Such a configuration reduces undesired ultrasonic echoes (noise) produced by reflection of the ultrasound waves in the wedge, which improves the defect detection accuracy.

Further, it is preferred that the partition absorber is spaced apart from a bottom surface of the wedge.

Such a configuration prevents the partition absorber from blocking the propagation of surface waves, which makes it possible to effectively detect the defect present in the surface area.

Further, it is preferred that the transmitting transducer is in the form of a broadband transducer. The broadband transducer refers to a transducer for generating an ultrasonic pulse containing one or two cycles.

Such a configuration makes it possible to detect, by means of the transmitting transducer and the receiving transducer, the defect present in the surface area of the test object. Specifically, since the number of cycles of the ultrasound transmitted from the broadband transducer is small, it is possible to discriminate between a reception signal that indicates reception by the receiving transducer of an ultrasonic echo produced by reflection of the ultrasound on the surface of the test object and a reception signal that indicates reception by the receiving transducer of an ultrasonic echo produced by reflection of the ultrasound by the defect present in the surface area. This enhances the reduction of surface dead zone.

## Claims

1. An ultrasonic probe, comprising:
a transmitting transducer for transmitting ultrasound;
a receiving transducer for receiving an ultrasonic echo produced by ultrasound transmitted from the transmitting transducer being reflected by a defect present in an internal area of a test object;
an absorber for absorbing ultrasound transmitted from the transmitting transducer;
a transmitting and receiving transducer including
a transmitting unit for transmitting ultrasound and
a receiving unit for receiving an ultrasonic echo produced by ultrasound transmitted from the transmitting unit being reflected by a defect present in a surface area of the test object; and
a wedge holding the transmitting transducer, the receiving transducer, the absorber, and the transmitting and receiving transducer, wherein
the wedge includes
a first holding part holding the transmitting transducer at an angle allowing the ultrasound transmitted from the transmitting transducer to penetrate into the test object,
a second holding part holding the receiving transducer at an angle allowing the receiving transducer to receive the ultrasonic echo produced by the ultrasound transmitted from the transmitting transducer being reflected by the defect present in the internal area,
an absorber holding part disposed between the first holding part and the second holding part and holding the absorber, and
a third holding part disposed on the opposite side of the first holding part from the absorber holding part and holding the transmitting and receiving transducer at an angle allowing the ultrasound transmitted from the transmitting and receiving transducer to propagate as surface waves along the surface area of the test object.

2. The ultrasonic probe according to claim 1, further comprising
a surface wave receiving transducer for receiving ultrasound produced by propagation of the surface waves into the wedge, wherein
the wedge further includes a fourth holding part disposed on the opposite side of the second holding part from the absorber holding part and holding the surface wave receiving transducer.

3. The ultrasonic probe according to claims 1 or 2, further comprising
a partition absorber, disposed between the transmitting transducer and the transmitting and receiving transducer, for absorbing part of the respective ultrasound waves transmitted from the transmitting transducer and the transmitting and receiving transducer.

4. The ultrasonic probe according to claim 3, wherein
the partition absorber is spaced apart from a bottom surface of the wedge.

5. The ultrasonic probe according to claim 1, wherein
the transmitting transducer is in the form of a broadband transducer.
